# FASCICULE DE BREVET EUROPEEN

(11) **EP 1 038 515 B1**
(45) Date de publication et mention de la délivrance du brevet: **14.07.2004**
(21) Numéro de dépôt: 00400495.8
(22) Date de dépôt: 23.02.2000
(51) Int. Cl.: A61K 7/06, A61K 7/48

(54) **Composition à phase aqueuse continue renfermant de l'acide L-2-oxothiazolidine 4-carboxylique**
Zusammensetzung mit kontinuierlicher wässeriger Phase, die L-2-Oxothiazolidin- 4-carbonsäure enthält".
Composition having an aqueous phase containing l-2-oxothiazolidine-4-carboxylic acid

(30) Priorité: 19.03.1999 FR 9903476
(43) Date de publication de la demande: 27.09.2000
(73) Titulaire: L'OREAL, 75008 Paris (FR)
(72) Inventeur: Chevalier, Véronique, 94400 Villecresnes (FR); Quest, Mélanie, 75005 Paris (FR)
(74) Mandataire: Renard, Emmanuelle

(56) Documents cités:
- EP-A- 0 650 725
- EP-A- 0 656 201
- EP-A- 0 780 120
- EP-A- 0 931 542
- FR-A- 2 773 323

## Description

La présente invention se rapporte à une composition à phase aqueuse continue renfermant de l'acide L-2-oxothiazolidine 4-carboxylique.

Il est connu d'utiliser l'acide L-2-oxothiazolidine 4-carboxylique, ou procystéine, dans des compositions cosmétiques ou dermatologiques destinées à une application topique sur la peau.

De telles compositions peuvent être destinées à prévenir la chute des cheveux ou à stimuler la repousse des cheveux, comme il est décrit dans EP-656 201.

La procystéine est également connue par le brevet FR-2 742 658 comme agent dépigmentant ou blanchissant de la peau humaine.

Cependant, la procystéine présente une certaine instabilité, en particulier lorsqu'elle est en présence d'eau. Aussi, lorsqu'elle est introduite dans une composition cosmétique, en particulier une composition comprenant de l'eau, son efficacité diminue au cours du temps. En outre, la composition dans laquelle elle est introduite, après un certain temps de stockage, présente des signes de dégradation : coloration, odeur, qui sont inacceptables pour l'utilisateur.

Une solution pour stabiliser la procystéine dans l'eau a consisté à l'associer avec un polyol, dans des quantités en poids particulières, et à limiter la quantité d'eau à une valeur de 35 % en poids, et de préférence inférieure à 20% en poids, par rapport au poids total de la composition.

Si ces compositions permettent effectivement de véhiculer la procystéine de façon stable au cours du temps sans perte d'efficacité de cet actif, leur texture ne convient pas nécessairement à tous les utilisateurs et il reste le besoin de disposer de compositions susceptibles de renfermer de grandes quantités d'eau, par exemple supérieures à 80% en poids, en particulier sous la forme d'émulsions huile-dans-eau.

Des compositions sous la forme d'émulsions huile-dans-eau renfermant de la procystéine sont certes connues de EP 656 201, mais la Demanderesse a constaté que ces compositions n'étaient pas stables au cours du temps et conduisaient à une dégradation de la procystéine, entraînant l'apparition d'une odeur soufrée nauséabonde et un jaunissement de la composition.

La Demanderesse a maintenant découvert que l'utilisation, en association, d'un séquestrant et d'un neutralisant en quantité suffisante pour ajuster le pH de la composition à une valeur comprise entre 5 et 8 permettait de formuler la procystéine dans des compositions à phase aqueuse continue qui restent stables au cours du temps et/ou à la température.

La présente invention a donc pour objet une composition à phase aqueuse continue comprenant de l'acide L-2-oxothiazolidine 4-carboxylique, au moins un séquestrant et au moins un neutralisant, caractérisée en ce que ledit neutralisant est présent en une quantité suffisante pour ajuster le pH de la phase aqueuse de la composition à une valeur comprise entre 5 et 8.

La composition obtenue est stable, notamment vis-à-vis de la dégradation thermique, y compris à des teneurs en eau allant jusqu'à 97% en poids, ce qui la rend particulièrement bien appropriée à des applications cosmétiques ou dermatologiques.

Avantageusement, la composition selon l'invention comprend de 0,01 à 10% en poids, préférentiellement de 0,1 à 5%, encore plus préférentiellement de 0,5 à 3% en poids d'acide L-2-oxothiazolidine 4-carboxylique par rapport au poids total de la composition.

Le neutralisant utilisé dans la composition selon l'invention peut être notamment choisi parmi : les hydroxydes de métaux alcalins, tels que l'hydroxyde de sodium et la potasse ; l'ammoniac ; et les bases organiques, telles que la mono-, la di- et la triéthanolamine, l'aminométhylpropanediol-1,3, la N-méthyl glucamine et les acides aminés basiques comme l'arginine et la lysine ; et leurs mélanges. De préférence, le neutralisant est la triéthanolamine.

Le neutralisant est présent en une quantité suffisante pour ajuster le pH de la phase aqueuse de la composition entre 5 et 8, de préférence entre 6 et 7. Pour ce faire, l'homme du métier ajustera facilement la quantité de neutralisant en fonction des acides, autres que l'acide L-2-oxothiazolidine 4-carboxylique, éventuellement présents dans la composition selon l'invention et en fonction de la nature du neutralisant. Par exemple, dans le cas où la composition selon l'invention ne renferme pas d'autre acide que l'acide L-2-oxothiazolidine 4-carboxylique et où le neutralisant utilisé est la triéthanolamine, le rapport pondéral du neutralisant à l'acide L-2-oxothiazolidine 4-carboxylique est généralement compris entre 0,7:1 et 1,3:1, mieux, entre 0,9:1 et 1,2:1. Selon une forme d'exécution préférée de l'invention, le rapport pondéral du neutralisant à l'acide L-2-oxothiazolidine 4-carboxylique est d'environ 1:1.

Le séquestrant associé au neutralisant peut être notamment choisi parmi : l'EDTA ; les sels d'EDTA, tels que les sels disodique et tétrasodique d'EDTA ou le sel dipotassique d'EDTA ; le cocoamphodiacétate disodique ; l'acide diéthylènetriamine pentacétique et ses sels, tels que le sel pentasodique de l'acide diéthylènetriamine pentacétique ; le sel trisodique de l'acide nitrilo-tri-acétique ; l'acide ascorbique ; le citrate trisodique ; l'acide étidronique et ses sels, tels que le sel tétrasodique de l'acide étidronique ; le sel heptasodique de l'acide diéthylènetriamine pentaméthylène phosphonique ; le sel pentasodique de l'acide diéthylènetriamine tétraméthylène phosphonique ; l'acide éthylènediamine tétraméthylène phosphonique et ses sels, tels que le sel pentasodique de l'acide éthylènediamine tétraméthylène phosphonique ; le glucoheptanoate de sodium ; et leurs mélanges. De préférence, le séquestrant est un sel d'EDTA.

Le séquestrant est par exemple présent en une quantité comprise entre 0,01 et 1% en poids, de préférence entre 0,01 et 0,5% en poids, mieux, en une quantité d'environ 0,05% en poids, par rapport au poids total de la composition.

La composition selon l'invention peut être plus ou moins fluide et avoir l'aspect d'une crème blanche ou colorée, d'un lait, d'une lotion, d'un sérum, d'une mousse ou d'un gel aqueux ou hydroalcoolique. Elle peut également se présenter sous la forme d'un stick aqueux ou hydroalcoolique. La composition peut éventuellement être appliquée sur la peau ou sur les cheveux sous forme d'aérosol.

Selon une forme d'exécution préfère, la composition selon l'invention se présente sous la forme d'une émulsion du type huile-dans-eau. Par cette expression, on entend généralement le mélange de deux liquides non miscibles dont l'un forme une phase huileuse discontinue et l'autre forme une phase aqueuse continue dans laquelle la phase huileuse est dispersée. En variante, toutefois, l'émulsion huile-dans-eau peut être constituée d'une dispersion d'huile dans une phase aqueuse à l'aide de sphérules, ces sphérules pouvant être des nanoparticules polymériques, telles que les nanosphères et les nanocapsules, ou des vésicules lipidiques de type ionique et/ou non-ionique, telles que celles décrites dans les demandes EP 641 557 et EP 705 593.

La composition selon l'invention peut être utilisée comme produit de soin et/ou comme produit de maquillage pour la peau. Elle peut également être sous une forme de shampooing ou d'après-shampooing.

Dans tous les cas, la composition comprend un milieu physiologiquement acceptable, c'est-à-dire compatible avec la peau ou ses phanères.

Lorsque la composition est sous la forme d'une émulsion huile-dans-eau, la proportion de la phase grasse discontinue peut représenter de 0,5 à 70%, et de préférence de 20 à 50%, du poids total de la composition. Les huiles, les émulsionnants et les coémulsionnants utilisés dans la composition sont choisis parmi ceux classiquement utilisés dans le domaine considéré.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras ou des cires (cire de carnauba, ozokérite).

Comme émulsionnants et coémulsionnants, on peut utiliser tout tensioactif permettant l'obtention d'une émulsion huile-dans-eau et par exemple : les esters d'acides gras de sorbitan, éventuellement polyoxyalkylénés, les esters d'acides gras de glycérol, les esters d'acides gras de polyalkylène glycol, les alcools gras, les éthers d'alcools gras polyoxyéthylénés, les sulfates de monoglycéryléthers, les alkyléthersulfates, les alkyl- ou alkényl-oligoglycosides, les N-alkyl polyhydroxyalkylamides d'acides gras, et leurs mélanges.

L'émulsionnant et le coémulsionnant sont présents, dans la composition, en une proportion allant de 0,3 à 30 % en poids, et de préférence de 0,5 à 20 % en poids par rapport au poids total de la composition. En variante, la composition selon l'invention peut être sous la forme d'une émulsion sans tensioactif, renfermant un polymère tel que celui disponible auprès de EASTMAN CHEMICAL sous la dénomination AQ38S. Ce polymère particulier est un polyester renfermant 89% molaire d'acide isophtalique, 11% molaire d'acide sodiosulfoisophtalique, 78% molaire de diéthylèneglycol et 22% molaire de 1,4-cyclohexane diméthanol.

De façon connue, la composition selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse (lorsqu'elle est présente), dans la phase aqueuse, dans les vésicules lipidiques et/ou dans les nanoparticules.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Comme actifs, on peut utiliser notamment les vitamines, les agents kératolytiques et/ou desquamants tels que l'acide n-octanoyl-5-salicylique, les filtres UV, les agents apaisants et leurs mélanges. On peut également incorporer à la composition selon l'invention d'autres agents dépigmentants, tels que l'acide kojique ou l'hydroquinone et ses dérivés, ce qui permet d'utiliser ces derniers à des doses moins élevées. On peut aussi incorporer à la composition selon l'invention d'autres agents anti-chute et/ou repousse des cheveux. En cas d'incompatibilité, ces actifs et/ou l'acide L-2-oxothiazolidine 4-carboxylique peuvent être incorporés dans des sphérules, notamment des vésicules ioniques ou non-ioniques et/ou des nanoparticules (nanocapsules et/ou nanosphères), de manière à les isoler les uns des autres dans la composition. En outre, on veillera à ce que les actifs éventuellement introduits dans la composition selon l'invention, et leurs concentrations, ne modifient pas les propriétés recherchées de l'acide L-2-oxothiazolidine 4-carboxylique.

Comme conservateurs, on pourra utiliser notamment les parabens et le phenoxyéthanol, mais on évitera de préférence les conservateurs donneurs de formol tels que la diazolydinyl urée.

L'invention a également pour objet l'utilisation d'au moins un séquestrant associé à au moins un neutralisant pour stabiliser l'acide L-2-oxothiazolidine 4-carboxylique dans une composition à phase aqueuse continue, ledit neutralisant étant présent en une quantité suffisante pour ajuster le pH de la phase aqueuse de la composition à une valeur comprise entre 5 et 8.

L'invention a en outre pour objet un procédé de stabilisation de l'acide L-2-oxothiazolidine 4-carboxylique dans une composition à phase aqueuse continue, ce procédé comprenant l'étape consistant à incorporer à ladite composition au moins un séquestrant et au moins un neutralisant, ledit neutralisant étant présent en une quantité suffisante pour ajuster le pH de la phase aqueuse de la composition à une valeur comprise entre 5 et 8.

La composition selon l'invention peut être utilisée, dans le domaine cosmétique, notamment aux fins suivantes :
- pour dépigmenter ou blanchir la peau, les poils et/ou les cheveux ;
- pour prévenir la chute des cheveux et/ou stimuler la repousse des cheveux ;
- pour prévenir ou traiter le photo-vieillissement et/ou le stress environnemental, en particulier en raison des propriétés anti-radicalaires de l'acide L-oxothiazolidine-4-carboxylique; et/ou
- pour prévenir ou traiter les peaux grasses, par exemple dans le traitement de l'acné.

Le stress environnemental est défini, dans cette demande, comme l'ensemble des facteurs chimiques et/ou physiques de dégradation de la peau liés à la vie urbaine et, plus précisément, à l'exposition de la peau aux fines particules de polluants flottant dans l'air et susceptibles de déclencher des réactions d'oxydation au contact de la peau. Ces fines particules peuvent être contenues par exemple dans la fumée de tabac, les gaz d'échappement ou les fumées industrielles. Il peut notamment s'agir de polluants tels que les oxydes d'azote et l'oxygène actif. La dégradation de l'état de la peau se traduit par l'un ou plusleurs des symptômes suivants : la formation de rides, une réduction de la teneur en eau de la peau, la formation de squames, une perte d'éclat du teint, des rougeurs, etc.

L'invention a également pour objet l'utilisation de la composition décrite ci-dessus pour la fabrication d'une préparation destinée à prévenir la chute des cheveux et/ou à stimuler la repousse des cheveux.

### EXEMPLES

Les exemples suivants sont donnés à titre d'illustration de l'invention. Les pourcentages sont donnés en poids par rapport au poids total de chaque constituant de la composition.

### Exemple 1

On prépare la composition suivante :

| | |
|---|---|
| Cyclohexasiloxane | 10 % |
| Huile d'abricot | 6 % |
| Glycérine | 5 % |
| Sel d'aluminium de l'amidon réticulé par l'anhydride octénylsuccinique | 3 % |
| Alcool stéarylique et ceteareth-20 | 2 % |
| Sesquistéarate de méthylglucose | 2 % |
| Triéthanolamine | 1,1 % |
| Acide L-2-oxothiazolidine 4-carboxylique | 1 % |
| Conservateurs | 0,6 % |
| Gomme de xanthane | 0,25 % |
| EDTA disodique | 0,05 % |
| Eau | 69 % |

L'émulsion huile-dans-eau obtenue a un pH de 6,6 et elle est stable pendant au moins 2 mois aux températures suivantes : 4°C, 25°C, 37°C et 45°C. On ne constate ni jaunissement, ni dégagement d'odeur, de la composition au bout de cette période de temps. En outre, le taux de dégradation de la procystéine, tel que déterminé par HPLC, est inférieur à 5%.

Cette composition peut être appliquée sur la peau matin et/ou soir pour atténuer les taches pigmentaires.

### Exemple 2 (comparatif)

On prépare une émulsion identique à celle de l'Exemple 1, excepté qu'elle ne contient pas de neutralisant (triéthanolamine).

L'émulsion obtenue a un pH de 2,4. Au bout de deux mois, elle dégage une odeur soufrée prononcée à toutes les températures (4°C, 25°C, 37°C et 45°C). On constate en outre une dégradation de 20% de l'acide L-2-oxothiazolidine 4-carboxylique pour l'émulsion conservée deux mois à 45°C.

### Exemple 3 (comparatif)

On prépare une émulsion identique à celle de l'Exemple 1, excepté qu'elle ne contient pas de séquestrant (EDTA disodique).

L'émulsion a un pH de 6,6. Au bout de deux mois, elle présente une légère odeur à toutes les températures (4°C, 25°C, 37°C et 45°C) et on constate en outre un léger jaunissement des émulsions conservées à 37°C et à 45°C.

### Exemple 4 (comparatif)

On prépare une émulsion identique à celle de l'Exemple 1, excepté qu'elle ne contient ni neutralisant (triéthanolamine), ni séquestrant (EDTA disodique).

L'émulsion a un pH de 2,3. Au bout de deux mois, elle présente une odeur soufrée prononcée et un jaunissement à toutes les températures (4°C, 25°C, 37°C et 45°C). On constate en outre une dégradation de 14% de l'acide L-2-oxothiazolidine 4-carboxylique pour l'émulsion conservée deux mois à 45°C.

Les exemples ci-dessus illustrent clairement la meilleure stabilité de la composition selon l'invention par rapport aux compositions ne comprenant pas de séquestrant et/ou de neutralisant.

### Exemple 5

On prépare la composition suivante :

| *Phase A* | |
|---|---|
| Alcool stéarylique | 1 % |
| Mélange de stéarate de glycéryle et de stéarate de polyéthylène glycol (100 OE) | 2 % |
| Cyclohexasiloxane | 10 % |

| *Phase B* | |
|---|---|
| Glycérine | 5 % |
| Hydroxyde de sodium | 0,01 % |
| EDTA | 0,05 % |
| Conservateurs | 0,2 % |
| Eau | qsp. |

| *Phase C* | |
|---|---|
| Gomme de xanthane | 0,2 % |
| Eau | 20 % |

| *Phase D* | |
|---|---|
| Acide L-oxothiazolidine-4-carboxylique | 1 % |
| Triéthanolamine | 1,1 % |
| Eau | 20 % |

| *Phase E* | |
|---|---|
| Amidon modifié octénylsuccinate | 3 % |
| Silice | 4 % |

La phase A et la phase B sont chauffées séparément à 80°C, puis la phase A est introduite dans la phase B sous agitation. Après refroidissement, on ajoute ensuite la phase C à l'émulsion obtenue, puis la phase D et enfin la phase E vers 40°C.

L'émulsion obtenue peut être utilisée pour réduire la brillance des peaux grasses.

## Revendications

1. Composition à phase aqueuse continue comprenant de l'acide L-2-oxothiazolidine 4-carboxylique, au moins un séquestrant et au moins un neutralisant, **caractérisée en ce que** ledit neutralisant est présent en une quantité suffisante pour ajuster le pH de la phase aqueuse de la composition à une valeur comprise entre 5 et 8.

2. Composition selon la revendication 1, **caractérisée en ce que** ledit séquestrant est choisi parmi : l'EDTA ; les sels d'EDTA, tels que les sels disodique et tétrasodique d'EDTA ou le sel dipotassique d'EDTA ; le cocoamphodiacétate disodique ; l'acide diéthylènetriamine pentacétique et ses sels, tels que le sel pentasodique de l'acide diéthylènetriamine pentacétique ; le sel trisodique de l'acide nitrilo-tri-acétique ; l'acide ascorbique ; le citrate trisodique ; l'acide étidronique et ses sels, tels que le sel tétrasodique de l'acide étidronique ; le sel heptasodique de l'acide diéthylènetriamine pentaméthylène phosphonique ; le sel pentasodique de l'acide diéthylènetriamine tétraméthylène phosphonique ; l'acide éthylènediamine tétraméthylène phosphonique et ses sels, tels que le sel pentasodique de l'acide éthylènediamine tétraméthylène phosphonique ; le glucoheptanoate de sodium ; et leurs mélanges.

3. Composition selon la revendication 2, **caractérisée en ce que** ledit séquestrant est un sel d'EDTA.

4. Composition selon l'une quelconque des revendications précédentes, **caractérisée en ce que** ledit neutralisant est choisi parmi : l'hydroxyde de sodium ; la potasse ; l'ammoniac ; et les bases organiques, telles que la mono-, la di- et la triéthanolamine, l'aminométhylpropanediol-1,3, la N-méthyl glucamine et les acides aminés basiques comme l'arginine et la lysine ; et leurs mélanges.

5. Composition selon la revendication 4, **caractérisée en ce que** ledit neutralisant est la triéthanolamine.

6. Composition selon l'une quelconque des revendications précédentes, **caractérisée en ce qu'**elle renferme en outre au moins un autre actif choisi parmi les vitamines, les agents dépigmentants, les agents kératolytiques et/ou desquamants, les agents apaisants et les filtres.

7. Composition selon l'une quelconque des revendications précédentes, **caractérisée en ce qu'**elle est sous la forme d'une émulsion huile-dans-eau.

8. Utilisation de la composition selon l'une quelconque des revendications 1 à 7 comme composition cosmétique destinée à dépigmenter ou blanchir la peau, les poils et/ou les cheveux.

9. Utilisation de la composition selon l'une quelconque des revendications 1 à 7 comme composition cosmétique destinée à prévenir la chute des cheveux et/ou à stimuler la repousse des cheveux.

10. Utilisation de la composition selon l'une quelconque des revendications 1 à 7 comme composition cosmétique destinée à prévenir ou traiter le photo-vieillissement et/ou le stress environnemental.

11. Utilisation de la composition selon l'une quelconque des revendications 1 à 7 comme composition cosmétique destinée à prévenir ou traiter les peaux grasses.

12. Utilisation de la composition selon l'une quelconque des revendications 1 à 7 pour la fabrication d'une préparation destinée à prévenir la chute des cheveux et/ou à stimuler la repousse des cheveux.

## Patentansprüche

1. Zusammensetzung mit kontinuierlicher wässeriger Phase, die L-2-Oxothiazolidin-4-carbonsäure, mindestens ein Maskierungsmittel und mindestens ein Neutralisationsmittel enthält, **dadurch gekennzeichnet, dass** das Neutralisationsmittel in einer Menge vorliegt, die ausreicht, um den pH-Wert der wässerigen Phase der Zusammensetzung auf einen Wert im Bereich von 5 bis 8 einzustellen.

2. Zusammensetzung nach Anspruch 1, **dadurch gekennzeichnet, dass** das Maskierungsmittel ausgewählt ist unter: EDTA, den Salzen von EDTA, wie dem Dinatriumsalz und dem Tetranatriumsalz von EDTA oder dem Dikaliumsalz von EDTA, Dinatriumcocoamphodiacetat, Diethylentriaminpentaessigsäure und ihren Salzen, wie dem Pentanatriumsalz von Diethylentriaminpentaessigsäure, dem Trinatriumsalz von Nitrilotriessigsäure, Ascorbinsäure, Trinatriumcitrat, Etidronsäure und ihren Salzen, wie dem Tetranatriumsalz von Etidronsäure, dem Heptanatriumsalz von Diethylentriamin-penta(methylenphosphonsäure), dem Pentanatriumsalz von Diethylentriamin-tetra(methylenphosphonsäure), Ethylendiamin-tetra(methylenphosphonsäure) und ihren Salzen, wie dem Pentanatriumsalz von Ethylendiamin-tetra(methylenphosphonsäure), Natriumglucoheptanoat und den Gemischen dieser Verbindungen.

3. Zusammensetzung nach Anspruch 2, **dadurch gekennzeichnet, dass** das Maskierungsmittel ein Salz von EDTA ist.

4. Zusammensetzung nach einem der vorhergehenden Ansprüche, **dadurch gekennzeichnet, dass** das Neutralisationsmittel ausgewählt ist unter: Natriumhydroxid, Kaliumhydroxid, Ammoniak, organischen Basen, wie Monoethanolamin, Diethanolamin und Triethanolamin, Aminomethyl-1,3-propandiol, N-Methylglucamin und basischen Aminosäuren, wie Arginin und Lysin, sowie den Gemischen dieser Verbindungen.

5. Zusammensetzung nach Anspruch 4, **dadurch gekennzeichnet, dass** das Neutralisationsmittel Triethanolamin ist.

6. Zusammensetzung nach einem der vorhergehenden Ansprüche, **dadurch gekennzeichnet, dass** sie außerdem mindestens einen weiteren Wirkstoff enthält, der unter den Vitaminen, depigmentierenden Wirkstoffen, keratolytischen und/oder abschuppenden Wirkstoffen, reizlindernden Wirkstoffen und Filtern ausgewählt ist.

7. Zusammensetzung nach einem der vorhergehenden Ansprüche, **dadurch gekennzeichnet, dass** sie in Form einer Öl-in-Wasser-Emulsion vorliegt.

8. Verwendung der Zusammensetzung nach einem der Ansprüche 1 bis 7 als kosmetische Zusammensetzung, die dafür vorgesehen ist, die Haut, das Körperhaar und/oder die Haare zu depigmentieren oder zu bleichen.

9. Verwendung der Zusammensetzung nach einem der Ansprüche 1 bis 7 als kosmetische Zusammensetzung, die dafür vorgesehen ist, dem Haarausfall vorzubeugen und/oder den Haarwuchs zu stimulieren.

10. Verwendung der Zusammensetzung nach einem der Ansprüche 1 bis 7 als kosmetische Zusammensetzung, die dafür vorgesehen ist, der lichtinduzierten Alterung und/ oder dem Umweltstress vorzubeugen oder diese zu behandeln.

11. Verwendung der Zusammensetzung nach einem der Ansprüche 1 bis 7 als kosmetische Zusammensetzung, die dafür vorgesehen ist, fettiger Haut vorzubeugen oder sie zu behandeln.

12. Verwendung der Zusammensetzung nach einem der Ansprüche 1 bis 7 zur Herstellung eines Präparats, mit dem dem Haarausfall vorgebeugt und/oder der Haarwuchs stimuliert werden soll.

## Claims

1. Composition with a continuous aqueous phase comprising L-2-oxothiazolidine-4-carboxylic acid, at least one sequestering agent and at least one neutralizing agent, **characterized in that** the said neutralizing agent is present in an amount which is sufficient to adjust the pH of the aqueous phase of the composition to a value of between 5 and 8.

2. Composition according to Claim 1, **characterized in that** the said sequestering agent is chosen from: EDTA; EDTA salts such as the disodium and tetrasodium salts of EDTA or the dipotassium salt of EDTA; disodium cocoamphodiacetate; diethylenetriamine pentaacetic acid and its salts, such as the pentasodium salt of diethylenetriamine pentaacetic acid; the trisodium salt of nitrilotriacetic acid; ascorbic acid; trisodium citrate; etidronic acid and its salts, such as the tetrasodium salt of etidronic acid; the heptasodium salt of diethylenetriamine pentamethylene phosphonic acid; the pentasodium salt of diethylenetriamine tetramethylene phosphonic acid; ethylenediamine tetramethylene phosphonic acid and its salts, such as the pentasodium salt of ethylenediamine tetramethylene phosphonic acid; sodium glucoheptanoate; and mixtures thereof.

3. Composition according to Claim 2, **characterized in that** the said sequestering agent is an EDTA salt.

4. Composition according to any one of the preceding claims, **characterized in that** the said neutralizing agent is chosen from: sodium hydroxide; potassium hydroxide; ammonia; organic bases such as monoethanolamine, diethanolamine, triethanolamine, aminomethyl-1,3-propanediol, N-methylglucamine and basic amino acids such as arginine and lysine; and mixtures thereof.

5. Composition according to Claim 4, **characterized in that** the said neutralizing agent is triethanolamine.

6. Composition according to any one of the preceding claims, **characterized in that** it also contains at least one other active agent chosen from vitamins, depigmenting agents, keratolytic agents and/or desquamating agents, calmants and screening agents.

7. Composition according to any one of the preceding claims, **characterized in that** it is in the form of an oil-in-water emulsion.

8. Use of the composition according to any one of Claims 1 to 7, as a cosmetic composition intended for depigmenting or bleaching the skin, body hairs and/or head hair.

9. Use of the composition according to any one of Claims 1 to 7, as a cosmetic composition intended for preventing hair loss and/or for stimulating regrowth of the hair.

10. Use of the composition according to any one of Claims 1 to 7, as a cosmetic composition intended for preventing or treating light-induced ageing and/or environment-related stress.

11. Use of the composition according to any one of Claims 1 to 7, as a cosmetic composition intended for preventing or treating greasy skin.

12. Use of the composition according to any one of Claims 1 to 7, for the manufacture of a preparation intended for preventing hair loss and/or for stimulating regrowth of the hair.
